# EUROPEAN PATENT APPLICATION

(11) **EP 4 137 100 A1**
(43) Date of publication of application: **22.02.2023**
(21) Application number: 22191447.6
(22) Date of filing: 22.08.2022
(51) Int. Cl.: A61F 2/64, A61F 2/70

(54) **ARTIFICIAL KNEE JOINT, BOTH-LEG ARTIFICIAL KNEE JOINT DEVICE, METHOD OF CONTROLLING BOTH-LEG ARTIFICIAL KNEE JOINT DEVICE, AND PROGRAM TO CONTROL BOTH-LEG ARTIFICIAL KNEE JOINT DEVICE**

(30) Priority: 20.08.2021 JP 2021134640
(71) Applicant: Nabtesco Corporation, Tokyo 102-0093 (JP)
(72) Inventor: Saito, Masakazu, Tokyo, 102-0093 (JP); Okuda, Masahiko, Tokyo, 102-0093 (JP)
(74) Representative: Grünecker Patent- und Rechtsanwälte PartG mbB

(57) **Abstract**

A both-leg artificial knee joint device (200) includes a first artificial knee joint (20A) worn on a first leg of a user, a second artificial knee joint (20B) worn on a second leg of the user and capable of communicating with the first artificial knee joint (20A), and a controller (50) structured to control at least one of the first artificial knee joint (20A) and the second artificial knee joint (20B) based on information received from the other artificial knee joint (20). The first artificial knee joint (20A) and the second artificial knee joint (20B) communicate via a communication device (300) communicable with the first artificial knee joint (20A) and the second artificial knee joint (20B).

## Description

### DESCRIPTION

The present invention relates to an artificial knee joint.

An artificial knee joint or a prosthetic limb worn by a person who has lost a leg due to an injury or a disease, in which the rotational resistance of the knee shaft is controlled in accordance with the phase of walking of the user, is known. In the Stance phase in which the prosthetic limb is in contact with the ground and is under load, the rotational resistance of the knee shaft is increased to prevent the knee from being bent under load. In the Swing phase in which the prosthetic limb leaves the ground and is swung, the rotational resistance of the knee shaft is decreased to bend the knee and prevent the prosthetic limb from touching the ground.

[Patent literature 1] JP2011-101807

The conventionally used artificial knee joint is mainly assumed to be worn on one leg, and adjustment and control of the rotational resistance of the knee shaft and the like are usually performed for each artificial knee joint. Therefore, when a user who has lost both legs wears two artificial knee joints on both legs, it is necessary to separately adjust and control the artificial knee joints for each leg, which is complicated.

The present invention has been made in view of such a situation, and an object of the present invention is to provide a both-leg artificial knee joint device capable of efficiently controlling artificial knee joints worn on both legs.

In order to solve the above problem, an artificial knee joint according to an aspect of the present invention is an artificial knee joint to be worn on one leg of a user, and includes a receiver capable of receiving information from the other artificial knee joint worn on the other leg of the user, and a controller structured to control the artificial knee joint based on information received from the other artificial knee joint.

In this aspect, the artificial knee joint worn on one leg of the user can be efficiently controlled based on the information received from the other artificial knee joint worn on the other leg of the user.

Another aspect of the present invention is a both-leg artificial knee joint device. The device includes a first artificial knee joint worn on a first leg of a user, a second artificial knee joint worn on a second leg of the user and capable of communicating with the first artificial knee joint, and a controller structured to control at least one of the first artificial knee joint and the second artificial knee joint based on information received from the other artificial knee joint.

Still another aspect of the present invention is also a both-leg artificial knee joint device. This device includes an inputter capable of inputting first information of a first artificial knee joint worn on a first leg of a user, a presenter that presents second information of a second artificial knee joint worn on a second leg of the user when the first information is input by the inputter, and a controller structured to control the first artificial knee joint based on the first information input by the inputter.

In this aspect, information on one artificial knee joint worn on one leg of the user can be efficiently input with reference to information on the other artificial knee joint worn on the other leg of the user.

Still another aspect of the present invention is a method of controlling a both-leg artificial knee joint device. The method is for controlling a both-leg artificial knee joint device including a first artificial knee joint worn on a first leg of a user and a second artificial knee joint worn on a second leg of the user and capable of communicating with the first artificial knee joint, the method including: transmitting information from one of the first artificial knee joint and the second artificial knee joint to the other; and controlling the other artificial knee joint based on information received from the one artificial knee joint.

Still another aspect of the present invention is also a method of controlling a both-leg artificial knee joint device. The method includes inputting first information of a first artificial knee joint worn on a first leg of a user, presenting second information of a second artificial knee joint worn on a second leg of the user when the first information is input in the inputting, and controlling the first artificial knee joint based on the first information input in the inputting.

Optional combinations of the aforementioned constituting elements, and implementations of the disclosure in the form of methods, apparatuses, systems, recording mediums, and computer programs may also be practiced as additional modes of the present invention.
Fig. 1 illustrates a schematic configuration of an artificial knee joint and a prosthetic limb constituting a both-leg artificial knee joint device;
Fig. 2 illustrates a schematic configuration of an artificial knee joint and a prosthetic limb constituting a both-leg artificial knee joint device;
Fig. 3 schematically shows transition of the knee angle θ, the thigh angle Ψ, and the shin angle Φ in accordance with the phase of walking of the user of the prosthetic limb;
Fig. 4 shows a cylinder and a control mechanism;
Fig. 5 illustrates a flow of oil during a bending motion or a stretching motion of the artificial knee joint;
Fig. 6 schematically shows functional blocks that provide bend control of the artificial knee joint;
Fig. 7 shows load lines L in the phases of walking of the user of the prosthetic limb as arrows or vectors;
Fig. 8 is a functional block diagram of a both-leg artificial knee joint device; and
Fig. 9 illustrates a screen example of application software for a both-leg artificial knee joint device executed by a general-purpose communication device.

The invention will now be described by reference to the preferred embodiments. This does not intend to limit the scope of the present invention, but to exemplify the invention.

The present invention relates to a both-leg artificial knee joint device that adjusts and controls two artificial knee joints worn on both legs of a user. In the present embodiment, firstly each artificial knee joint or a prosthetic limb will be described, and then a both-leg artificial knee joint device including two artificial knee joints will be described. The configurations of each artificial knee joint in the both-leg artificial knee joint device are preferably the same or similar, but may be different.

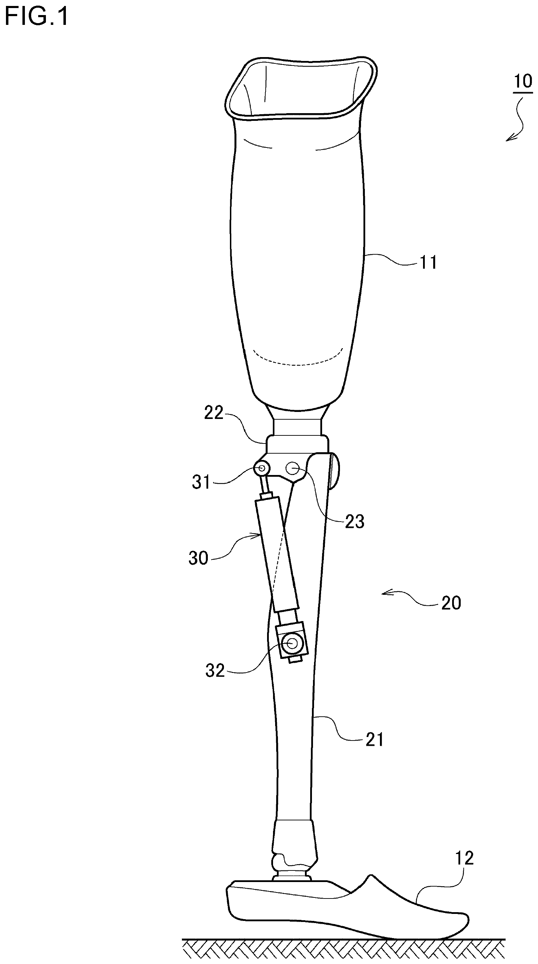

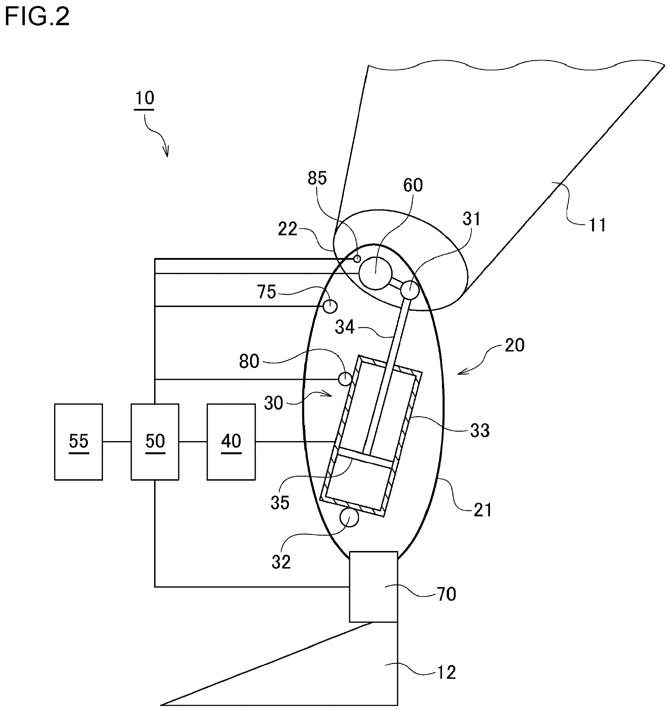

Figs. 1 and 2 show a schematic configuration of an artificial knee joint 20 and a prosthetic limb 10 constituting the both-leg artificial knee joint device according to the present invention. The prosthetic limb 10 is provided with a plastic socket 11 as a thigh part, a thigh joint part 22 to which the socket 11 is connected, a lower leg part 21 coupled to the thigh joint part 22 and provided to be rotatable around a knee shaft 23, and a foot part 12 coupled to the lower end of the lower leg part 21. The thigh joint part 22 to which the socket 11 is connected and the lower left part 21 bend or stretch the artificial knee joint 20 corresponding to the knee joint by relatively rotating around the knee shaft 23 provided in the joint between the thigh joint part 22 and the lower leg part 21 and perpendicular to the plane of Fig. 1. Further, the foot part 12 is formed by an elastic member, and the relative posture under a small load from the ground (e.g., when the foot part 12 does not touch the ground or when the user stands upright) is maintained constant by elasticity. Further, the elastic member is elastically deformed under a heavy load from the ground (e.g., when the user is walking) to produce a propelling power to kick the ground.

The artificial knee joint 20 is provided with the lower leg part 21 formed by a high-strength frame, the thigh joint part 22 connected to the socket 11 as a thigh part and coupled to the lower leg part 21 so as to be rotatable around the knee shaft 23, a cylinder 30 that restricts or permits the rotational motion around the knee shaft 23, i.e., the bending and stretching motion of the artificial knee joint 20, and a control mechanism 40 for driving the cylinder 30. Fig. 1 shows the artificial knee joint 20 in which the thigh joint part 22 and the lower leg part 21 are joined by a single link and which is rotatable around the single knee shaft 23 at a particular position. The present invention is equally applicable to the artificial knee joint 20 in which the thigh joint part 22 and the lower leg part 21 are joined by, for example, two front and rear links, and which is rotatable around the knee shaft 23 as a rotational center virtually and instantaneously formed inward of the total of four joints.

The amount of extension/contraction of the cylinder 30 and the knee angle, which is a rotational angle of the artificial knee joint 20 around the knee shaft 23 are substantially in one-to-one correspondence. A knee angle sensor 60 as a knee angle acquisition unit for measuring the amount of extension/contraction of the cylinder 30 to detect the knee angle of the artificial knee joint 20 is provided in the vicinity of the cylinder 30 and the thigh joint part 22. The knee angle detected by the knee angle sensor 60 is used by a controller 50. The knee angle sensor 60 may be formed by an arbitrary sensor capable of measuring the amount of extension/contraction of the cylinder 30. For example, the knee angle sensor 60 may be formed by a Hall device capable of sensing the position of a magnet embedded in a piston rod 34 that moves in association with the extension/contraction of the cylinder 30. The knee angle is an angle formed by the axis of the socket 11 as the thigh part and the axis of the lower leg part 21. As shown in Fig. 1, for example, the knee angle formed when the user of the prosthetic limb 10 is standing upright and the axis of the socket 11 and the axis of the lower leg part 21 are aligned is 0 degrees. Further, the knee angle, formed when the user of the prosthetic limb 10 is seated, the axis of the lower leg part 21 remains aligned in the vertical direction of Fig. 1, and the axis of the socket 11 changes to the horizontal direction, is 90 degrees.

An inertial sensor 75 provided in the lower leg part 21 senses the posture and motion of the lower leg part 21 by measuring the speed (angular speed) and/or acceleration (angular acceleration) in the translational direction and/or rotational direction of the three axes defining the motion of the lower leg part 21. As described later, the shin angle, which defines the posture of the lower leg part 21 sensed by the inertial sensor 75 and which is an inclination angle formed by the axis of the lower leg part 21 relative to the vertical line passing through the knee shaft 23, is used to control the artificial knee joint 20 according to the embodiment. The shin angle and the thigh angle, which is described later, can be sensed in two directions including the longitudinal direction and the transversal direction and can be used to control the artificial knee joint 20. Hereinafter, the terms shin angle and thigh angle shall refer to an inclination angle in the longitudinal direction unless otherwise specified. The inertial sensor 75 capable of sensing the shin angle forms a lower leg part inclination angle acquisition unit. The inertial sensor 75 can be provided at an arbitrary position of the lower leg part 21. For example, the inertial sensor 75 is implemented on a control board provided in the outer circumference of the cylinder 30 along with the controller 50.

The thigh angle, which is an inclination angle formed by the axis of the socket 11 as the thigh part relative to the vertical line passing through the knee shaft 23, can be computed from the knee angle acquired by the knee angle sensor 60 and the shin angle acquired by the inertial sensor 75. In other words, the shin angle is the inclination of the lower leg part 21 from the vertical line passing through the knee shaft 23, and the knee angle is the inclination of the axis of the socket 11 from the axis of the lower leg part 21. By totaling the two, therefore, the thigh angle, which is the inclination of the axis of the socket 11 from the vertical line passing through the knee shaft 23, can be computed. Therefore, the knee angle sensor 60 and the inertial sensor 75 form a thigh part inclination angle acquisition unit for acquiring the thigh angle. Alternatively, an inertial sensor may be provided in the socket 11 or the thigh joint part 22 to measure the thigh angle directly. In this case, the shin angle can be computed based on the knee angle and the thigh angle measured. Similarly, even if the knee angle sensor 60 is not provided, the knee angle can be computed by measuring the thigh angle and the shin angle.

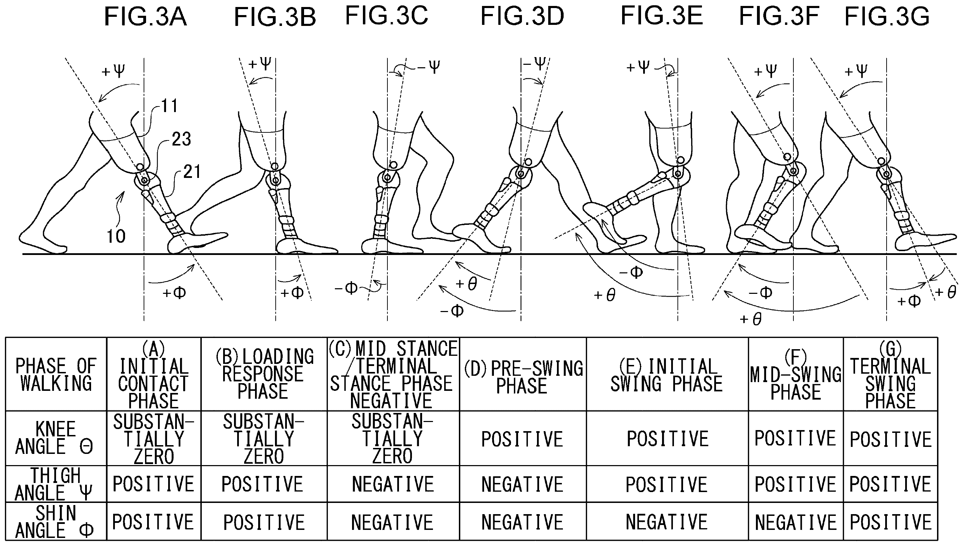

Fig. 3 schematically shows a transition of the knee angle θ, the thigh angle Ψ, and the shin angle Φ in accordance with the phase of walking (A)-(G) of the user of the prosthetic limb 10. The phase of walking (A) is the Initial Contact (IC) phase in which the prosthetic limb 10 touches the ground. The phase of walking (B) is the Loading Response (LR) phase in which the weight of the user is supported by the prosthetic limb 10 touching the ground. The phase of walking (C) is the Mid Stance (MSt) to Terminal Stance (TSt) phase during which the gravitational center of the user moves ahead of the prosthetic limb 10 while the prosthetic limb 10 is supporting the weight. The phase of walking (D) is the Pre-Swing (PSw) phase in which the prosthetic limb 10 starts kicking the ground to make a transition to the subsequent Swing phase. The phases of walking (E), (F), and (G) are the Initial Swing (ISw), Mid Swing (MSw), and Terminal Swing (TSw) phases, respectively, in which the prosthetic limb 10 having left the ground is swung from back to front.

The thigh angle Ψ is defined to be negative (illustrated as -Ψ) when the socket 11 is inclined forward around the knee shaft 23 with reference to the vertical line passing through the knee shaft 23 and is defined to be positive (illustrated as +Ψ) when the socket 11 is inclined rearward. The shin angle Φ is defined to be positive (illustrated as +Φ) when the lower leg part 21 is inclined forward around the knee shaft 23 with reference to the vertical line passing through the knee shaft 23 and is defined to be negative (illustrated as -Φ) when the lower leg part 21 is inclined rearward. The knee angle θ is defined to be positive (illustrated as +θ) when the lower leg part 21 is inclined rearward around the knee shaft with reference to the axis of the socket 11. The knee angle θ, the thigh angle Ψ, and the shin angle Φ defined as described above always meet the relational expression "θ+Φ=Ψ".

As illustrated, the knee angle θ is substantially zero/the thigh angle Ψ is positive/the shin angle Φ is positive in the Initial Contact phase (A), the knee angle θ is substantially zero/the thigh angle Ψ is positive/the shin angle Φ is positive in the Loading Response phase (B), the knee angle θ is substantially zero/the thigh angle Ψ is negative/the shin angle Φ is negative in the Mid Stance/Terminal Stance phase (C), the knee angle θ is positive/the thigh angle Ψ is negative/the shin angle Φ is negative in the Pre-Swing phase (D), the knee angle θ is positive/the thigh angle Ψ is positive/the shin angle Φ is negative in the Initial Swing phase (E), the knee angle θ is positive/the thigh angle Ψ is positive/the shin angle Φ is negative in the Mid Swing phase (F), and the knee angle θ is positive/the thigh angle Ψ is positive/the shin angle Φ is positive in the Terminal Swing phase (G).

To focus on the knee angle θ, the knee angle θ increases and decreases monotonically such that the knee angle θ is substantially zero in the Stance phases (A)-(C) in which the prosthetic limb 10 in contact the ground supports the weight of the user, the knee angle θ maintains a value from substantially zero to positive in the Pre-Swing phase (D) between the Stance phases (A)-(C) and the Swing phases (E)-(G), and has a maximum value in the Mid-Swing phase (F) of the Swing phases (E)-(G), in which the prosthetic limb 10 leaves the ground and is swung.

Control of the knee angle θ like this, i.e., bend control of the artificial knee joint 20, is performed by the controller 50 via the control mechanism 40 and the cylinder 30. In other words, the knee angle θ is maintained to be substantially zero in the Stance phases (A)-(C) by increasing the hydraulic resistance of the cylinder 30 and restricting the rotation around the knee shaft 23 to prevent the artificial knee joint 20 from being bent by the weight of the user. In the Pre-Swing phase (D), the hydraulic resistance of the cylinder 30 is reduced to permit the rotation around the knee shaft 23 so as to make it possible for the artificial knee joint 20 to start bending in preparation for the subsequent Swing phases (E)-(G), which ensures that the knee angle θ has a positive value. In the Swing phases (E)-(G), the hydraulic resistance of the cylinder 30 is maintained to be low to allow the artificial knee joint 20 to bend easily so that the prosthetic limb 10 swung away from the ground does not contact the ground. Details of bend control of the artificial knee joint 20 will be described later.

A description of the prosthetic limb 10 will be continued by referring back to Fig. 1 and Fig. 2. A load sensor 70 for detecting the load exerted between the lower leg part 21 and the foot part 12 may be provided at the lower end of the lower leg part 21, as a sensor other than the knee angle sensor 60 and the inertial sensor 75. Since it is possible to recognize the phase of walking of the user of the prosthetic limb 10 based on the magnitude and direction of the load detected by the load sensor 70, the load sensor 70 may be used for bend control of the artificial knee joint 20 in addition to, or instead of the knee angle sensor 60 and the inertial sensor 75. The load sensor may be provided in the thigh joint part 22 or in the knee part between the thigh joint part 22 and the lower leg part 21. Meanwhile, as described later, bend control of the artificial knee joint 20 can be performed by using the knee angle sensor 60 and the inertial sensor 75 only so that the load sensor 70 may be omitted to configure the artificial knee joint 20 inexpensively. Further, a temperature sensor 80 for measuring the temperature of the oil in the cylinder 30 is attached to the outer wall or the inner wall of the cylinder 30. The measurement information of these sensors is used in the controller 50.

A vibrator 85 is provided in the thigh joint part 22 or the lower leg part 21. The vibrator 85 notifies or alerts the user wearing the prosthetic limb 10 by vibration and is controlled by the controller 50.

The controller 50 controls the control mechanism 40 based on the measurement information of various sensor such as the knee angle sensor 60, the load sensor 70, the inertial sensor 75, and the temperature sensor 80 to control the resistance to the extension/contraction motion of the cylinder 30, i.e., the rotational resistance of the knee shaft 23 during the bending motion of the artificial knee joint 20. A battery 55 for supplying power to the parts of the artificial knee joint 20 is connected to the controller 50. Fig. 2 shows the control mechanism 40, the controller 50, and the battery 55 outside the artificial knee joint 20, but in practice are provided inside the lower leg part 21 as components constituting the artificial knee joint 20.

The cylinder 30 is a hydraulic cylinder that restricts or permits the bending or stretching motion of the artificial knee joint 20 by producing a drag by using oil as a working fluid. The cylinder 30 is supported by an upper support point 31 provided in the vicinity of the knee shaft 23 that rotatably couples the socket 11 and the lower leg part 21 and by a lower support point 32 coupled to a part of the lower leg part 21 and can extend or contract between the support points. In the compression step in which the cylinder length is reduced, a bending motion in which the artificial knee joint 20 is rotated counterclockwise of Fig. 1 around the knee shaft 23 is performed. In the expansion step in which the cylinder length is enlarged, a stretching motion in which the artificial knee joint 20 is rotated clockwise of Fig. 1 around the knee shaft 23 is performed. The cylinder length refers to a length between the upper support point 31 and the lower support point 32 of the cylinder 30.

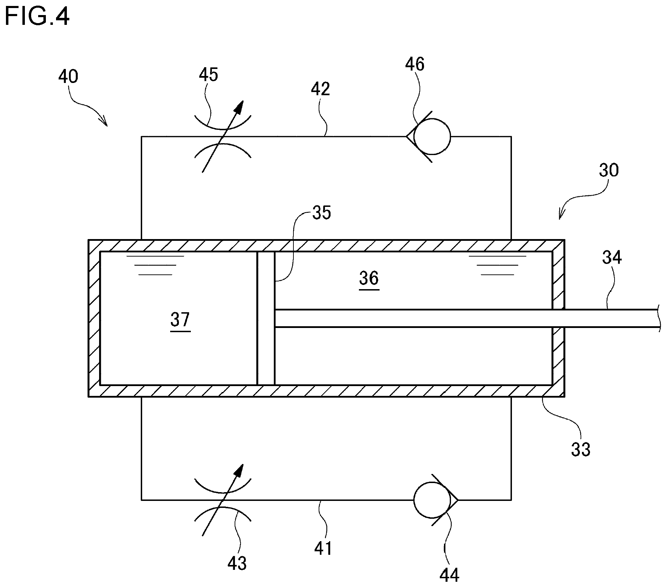

A description will now be given of the cylinder 30 and the control mechanism 40 with reference to Fig. 4. The cylinder 30 is provided with a cylinder tube 33, a piston rod 34 inserted from the side of one end (the right end side of Fig. 4) of the cylinder tube 33 and movable along the longitudinal direction (the transversal direction of Fig. 4) of the cylinder tube 33, and a piston 35 secured to the piston rod 34 in the cylinder tube 33 and slidable in the longitudinal direction along the inner wall of the cylinder tube 33. The piston 35 partitions the interior of the cylinder tube 33 into a first cavity 36 on the side of the one end (the right end side of Fig. 4) and a second cavity 37 on the side of the other end (the left end side of Fig. 4). The first cavity 36 and the second cavity 37 are filled with oil as a working fluid.

The control mechanism 40 is a hydraulic driving mechanism for driving the cylinder 30 into extension or contraction by a hydraulic pressure. The control mechanism 40 is provided with a stretching side hydraulic circuit 41 and a bending side hydraulic circuit 42 respectively connected to the cylinder 30. The stretching side hydraulic circuit 41 and the bending side hydraulic circuit 42 respectively communicate with the first cavity 36 at one end and communicate with the second cavity 37 at the other end. The stretching side hydraulic circuit 41 is provided with a stretching side valve 43 as a valve capable of opening or closing the fluid path of the oil for producing the rotational resistance of the knee shaft 23 and with a stretching side check valve 44. Opening the stretching side valve 43 allows the oil to be distributed in the stretching side hydraulic circuit 41. The action of the stretching side check valve 44 causes the oil to flow only in the direction from the first cavity 36 to the second cavity 37 and not to flow in the opposite direction.

The bending side hydraulic circuit 42 is provided with a bending side valve 45 as a valve capable of opening or closing the fluid path of the oil for producing the rotational resistance of the knee shaft 23 and with a bending side check valve 46. Opening the bending side valve 45 allows the oil to be distributed in the bending side hydraulic circuit 42. The action of the bending side check valve 46 causes the oil to flow only in the direction from the second cavity 37 to the first cavity 36 and not to flow in the opposite direction. The positions of the stretching side valve 43 and the bending side valve 45 are individually controlled by the controller 50. The position of each valve can have an arbitrary value between fully open (the most open position) and fully closed (the least open position). When the valve is fully closed, the flow of the oil is interrupted so that the hydraulic pressure is maximized. The more open the valve toward full open, the larger the cross section through which the oil can be distributed in the valve and the smaller the hydraulic resistance.

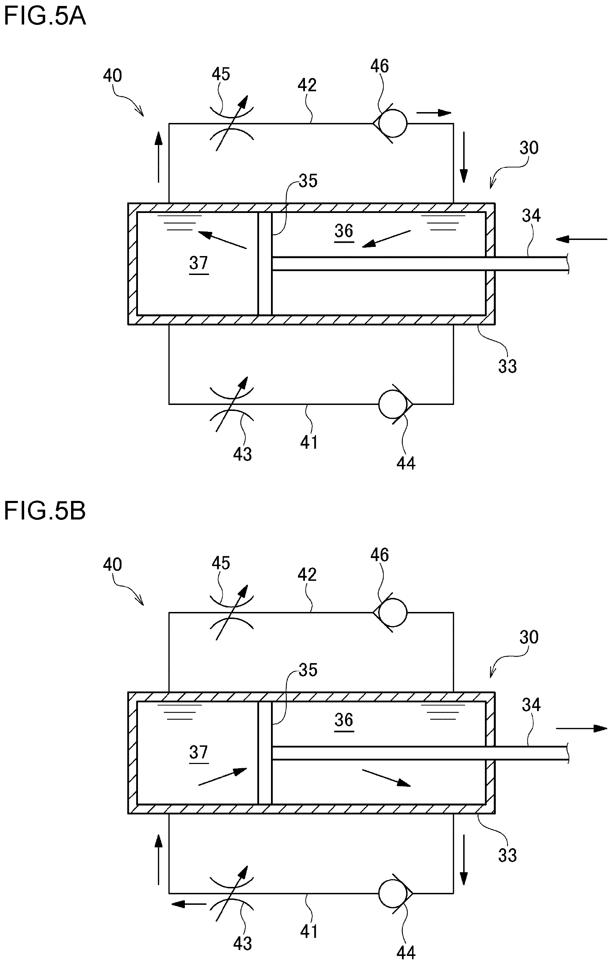

Fig. 5A shows the flow of the oil in the bending motion of the artificial knee joint 20. Bending is a compression step in which the cylinder length is decreased. The piston rod 34 recedes leftward in Fig. 5, and the piston 35 moves toward the intake side. The oil extruded from the second cavity 37 by the movement of piston 35 cannot be distributed in the stretching side hydraulic circuit 41 provided with the stretching side check valve 44 and so flows into the first cavity 36 via the bending side hydraulic circuit 42. Controlling the bending side valve 45 to be less open in this process makes it difficult for the oil to flow in the bending side hydraulic circuit 42 and so can restrict the bending motion of the artificial knee joint 20. Thus, the controller 50 constitutes the rotational resistance controller for controlling the rotational resistance of the knee shaft 23 during the bending motion of the artificial knee joint 20, by controlling the position of the bending side valve 45.

Fig. 5B shows the flow of the oil in the stretching motion of the artificial knee joint 20. Stretching is an expansion step in which the cylinder length is increased. The piston rod 34 advances rightward in Fig. 5, and the piston 35 moves toward the extrusion side. The oil extruded from the first cavity 36 by the movement of piston 35 cannot be distributed in the bending side hydraulic circuit 42 provided with the bending side check valve 46 and so flows into the second cavity 37 via the stretching side hydraulic circuit 41. Controlling the stretching side valve 43 to be less open in this process makes it difficult for the oil to flow in the stretching side hydraulic circuit 41 and so can restrict the stretching motion of the artificial knee joint 20. Thus, the controller 50 constitutes the rotational resistance controller for controlling the rotational resistance of the knee shaft 23 during the stretching motion of the artificial knee joint 20, by controlling the position of the stretching side valve 43.

Reference is made back to Fig. 2, and the sensors provided in the prosthetic limb 10 will be described further.

The knee angle sensor 60 measures the position of the piston rod 34 extended or contracted. For example, the position of a magnet attached to the piston rod 34 is measured by a magnetic sensor provided in the cylinder tube 33. The position of the piston rod 34 extended or contracted and the knee angle of the artificial knee joint 20 or the rotational angle of the knee shaft 23 are in one-to-one correspondence. Therefore, the knee angle sensor 60 can convert the detected position of the piston rod 34 extended or contracted into the knee angle of the artificial knee joint 20. The computation to convert the position of the piston rod 34 extended or contracted into the knee angle of the artificial knee joint 20 may be performed in the controller 50. The knee angle sensor 60 in this case measures the position of the piston rod 34 extended or contracted and provides the measurement to the controller 50.

The inertial sensor 75 senses the posture and motion of the lower leg part 21 based on the measured speed and/or acceleration of the respective axes. For example, the phase of walking of the user of the prosthetic limb 10 can be recognized from the variation in the speed/acceleration measured by the inertial sensor 75, and the position of the lower leg part 21 during waling can be tracked by integrating measurement values of the inertial sensor 75. Therefore, the step length, which is an amount of displacement per step, and the walking speed per step can be determined. The computation to determine the position by integrating measurement values of the inertial sensor 75, etc. is performed by the controller 50.

The load sensor 70 is comprised of, for example, a strain sensor and is provided in the ankle part between the lower leg part 21 and the foot part 12. The load exerted on the ankle part produces a strain in the object that constitutes the strain sensor so that the load can be measured by detecting the strain. By providing such a load sensor in the thigh joint part 22, the load applied on the knee joint can also be measured. The computation to convert the strain detected by the strain sensor into the load may be performed by the controller 50. The load sensor 70 in this case provides the detected strain to the controller 50. Further, the controller 50 can recognize the phase of walking of the user of the prosthetic limb 10 from the variation in the magnitude and direction of the load measured by the load sensor 70 and so can determine the step length and the walking speed by computation. Also, information on the moment exerted on the artificial knee joint 20 can be obtained from the position, magnitude, direction of the load measured by the load sensor 70.

The temperature sensor 80 measures the temperature of the cylinder 30 or the temperature of the oil in the cylinder 30. For example, the temperature sensor 80 initiates a transition to a high-temperature mode in which the action of the artificial knee joint 20 is restricted upon detecting that the cylinder 30 reaches a high temperature due to the heat from the hydraulic resistance. Further, although the hydraulic resistance varies in accordance with the temperature variation depending on the physical property of the oil, the controller 50 can realize a desired hydraulic resistance by controlling the control mechanism 40 in accordance with the temperature measured by the temperature sensor 80. More specifically, control data set for the control mechanism 40 to realize the respective values of hydraulic resistance is created for each temperature and stored in the controller 50. The controller 50 selects the control data set corresponding to the temperature measured by the temperature sensor 80 and uses the data for control.

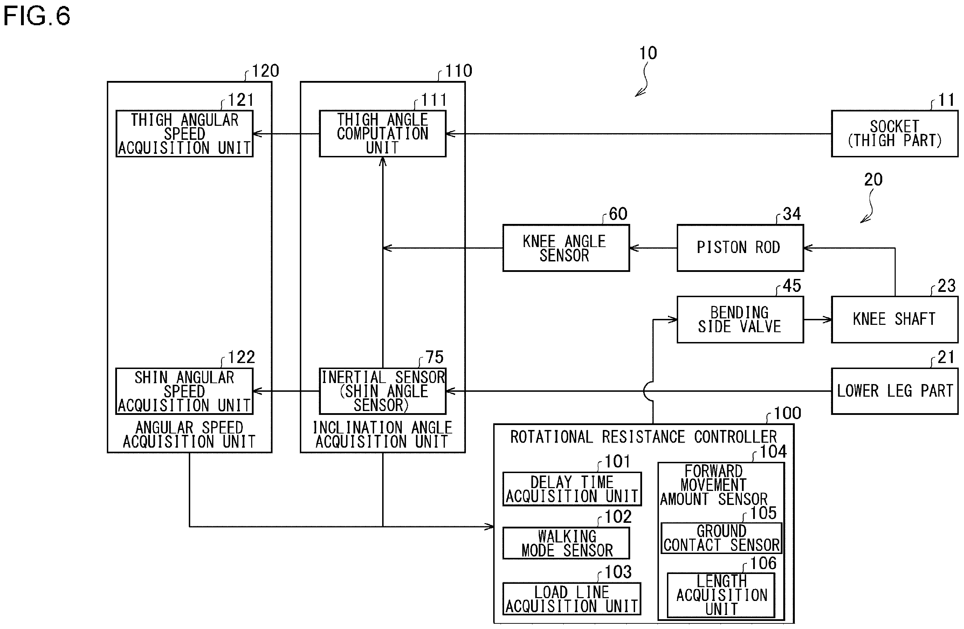

Fig. 6 schematically shows functional blocks that provide bend control of the artificial knee joint 20. The figure shows only those constituting elements of the prosthetic limb 10 related to bend control of the artificial knee joint 20. Further, the illustrated constituting elements are provided in the artificial knee joint 20 except for the socket 11. The constituting elements that provide information processing such as a rotational resistance controller 100 are implemented in the controller 50.

An inclination angle acquisition unit 110 is provided with a thigh angle computation unit 111 that acquires the thigh angle Ψ, which is an inclination angle formed by the socket 11 as the thigh part relative to the vertical line passing through the knee shaft 23, constantly or at a predetermined time interval and with the inertial sensor 75 as a shin angle sensor that acquires the shin angle Φ, which is an inclination angle formed by the lower leg part 21 relative to the vertical line passing through the knee shaft 23, constantly or at a predetermined time interval. As described above, the knee angle θ, the thigh angle Ψ, and the shin angle Φ meet the relational expression "θ+Φ=Ψ" so that the thigh angle computation unit 111 can compute the thigh angle Ψ=θ+Φ based on the knee angle θ measured by the knee angle sensor 60 and the shin angle Φ measured by the inertial sensor 75.

An angular speed acquisition unit 120 is provided with a thigh angular speed acquisition unit 121 (a thigh part inclination angular speed acquisition unit) that acquires the angular speed of the thigh angle Ψ and a shin angular speed acquisition unit 122 that acquires the angular speed of the shin angle Φ. More specifically, the thigh angular speed acquisition unit 121 determines the thigh angular speed by differentiating the thigh angle Ψ computed by the thigh angle computation unit 111 with respect to time, and the shin angular speed acquisition unit 122 determines the shin angular speed by differentiating the shin angle Φ measured by the inertial sensor 75 with respect to time. When the inertial sensor 75 as a shin angle sensor can measure the shin angular speed directly, the inertial sensor 75 itself constitutes the shin angular speed acquisition unit 122. Alternatively, the thigh angular speed may be computed based on the shin angular speed acquired by the shin angular speed acquisition unit 122 and the knee angular speed determined by differentiating the knee angle θ measured by the knee angle sensor 60 with respect to time.

The rotational resistance controller 100 controls the rotational resistance (hereinafter, bend resistance) of the knee shaft 23 during the bending motion of the artificial knee joint 20, by controlling the position of the bending side valve 45. The rotational resistance controller 100 is provided with, as constituting elements for acquiring various information used for control of bend resistance, a delay time acquisition unit 101, a walking mode sensor 102, a load line acquisition unit 103, and a forward movement amount sensor 104. The rotational resistance controller 100 need not be provided with all of these functional units but may be provided with at least one of the function units.

The delay time acquisition unit 101 acquires a delay time that elapses until the result of control of the bending side valve 45 by the rotational resistance controller 100 is reflected in the actual bend resistance. The delay time is primarily determined by the control period of the rotational resistance controller 100 and by the driving period during which the motor is driven to open or close the bending side valve 45 to a desired position. Given, for example, that the control period of the rotational resistance controller 100 is 50 ms and the driving period of the motor is 50 ms, a delay time of about 100 ms in total is produced. As described later, the bend resistance is controlled in the embodiment by allowing for the delay time as well.

The walking mode sensor 102 senses the walking mode of the user wearing the prosthetic limb 10 based on the posture and motion of the respective parts of the prosthetic limb 10 that can be sensed by the knee angle sensor 60, the inertial sensor 75, the thigh angle computation unit 111, the angular speed acquisition unit 120, etc. For example, the walking mode sensor 102 can sense that the user of the prosthetic limb 10 is walking forward ordinarily as shown in Fig. 3 and can sense the phases of walking (A)-(G) during the walk. The walking mode sensor 102 can also sense a particular walking mode different from the ordinary walking mode of Fig. 3. For example, the walking mode sensor 102 can sense an abnormal walk mode such as backward walking and a forward, backward, leftward, rightward, or upward jump.

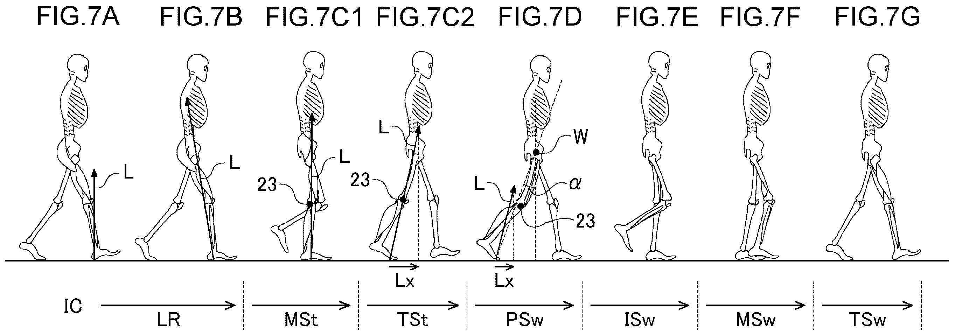

The load line acquisition unit 103 acquires a load line L indicating the direction of load exerted on the lower leg part 21. Fig. 7 shows load lines L in the phases of walking, equally shown in Fig. 3, as arrows or vectors. The length of the load line L represents the magnitude of load. The phases of walking (A)-(G) of Fig. 7 correspond to the phases of walking (A)-(G) of Fig. 3, but the Mid Stance/Terminal Stance phase shown as one phase of walking (C) in Fig. 3 is divided into two phases of walking, i.e., the Mid Stance (MSt) phase (C1) and the Terminal Stance (TSt) phase (C2), in Fig. 7.

To focus on the relationship between the load line L and the knee shaft 23 in the phases of walking (C1)-(D), the knee shaft 23 (the virtual and instantaneous rotational center in the artificial knee joint 20 in which the thigh joint part 22 and the lower leg part 21 are joined by a plurality of links) is slightly behind the load line L in the Mid Stance phase (C1). The knee shaft 23 is on the load line L in the Terminal Stance phase (C2), and the knee shaft 23 is in front of the load line L in the Pre-Swing phase (D). Thus, the knee shaft 23 makes a transition from a point behind the load line L to a point in front of the load line L, in association with the transition between the phases of walking (C1)-(D). Like the walking mode described above, the load line L is determined based on the posture and motion of the respective parts of the prosthetic limb 10 that can be sensed by the inertial sensor 75, the thigh angle computation unit 111, the angular speed acquisition unit 120, etc. Further, the load line L can be measured directly by the sensor 70 when the load sensor 70 is provided in the artificial knee joint 20.

The forward movement amount sensor 104 senses the amount of forward movement of the user wearing the artificial knee joint 20. As shown in the Terminal Stance phase (C2) and the Pre-Swing phase (D) of Fig. 7, the component Lx of the load line L in the horizontal direction or the direction of travel is the forward movement amount, which represents an advancing force from the load that contributes to the advancement of the user of the prosthetic limb 10. It is sufficient if the variation or increase/decrease of the forward movement amount is known for control of bend resistance by the rotational resistance controller 100. It is not necessary to sense the forward movement amount or the advancing force strictly. Therefore, it is sufficient for the forward movement amount sensor 104 to acquire data correlated to the forward movement amount.

Data indicative of the forward movement amount is exemplified by the thigh angular speed acquired by the thigh angular speed acquisition unit 121. In association with the occurrence of a forward movement amount in the Terminal Stance phase (C2) and the Pre-Swing phase (D) of Fig. 7, the thigh angular speed has a negative value in the Mid Stance/Terminal Stance phase (C) and the Pre-Swing phase (D) of Fig. 3. Therefore, a negative thigh angle speed suggests a forward movement amount.

Data indicative of the forward movement amount can also be obtained from a ground contact sensor 105 or a length acquisition unit 106. The ground contact sensor 105 senses the contact of the artificial knee joint 20 with the ground. The length acquisition unit 106 acquires the length of the artificial knee joint 20 from the thigh joint part 22 to the lower end of the lower leg part 21. In the Terminal Stance phase (C2) and the Pre-Swing phase (D) in which a forward movement amount occurs in Fig. 7, the leg wearing the artificial knee joint 20 is in contact with the ground, and the line connecting the toe and the waist W is inclined forward. It can therefore be said that a forward movement amount is present when the angle α of forward inclination of the waist W has increased relative to the vertical line when the ground contact sensor 105 senses that the artificial knee joint 20 is in contact with the ground.

When the artificial knee joint 20 is provided with the load sensor 70 as a load sensor, the ground contact sensor 105 can directly sense that the artificial knee joint 20 is in contact with the ground by referring to the load measured by the load sensor 70. However, it might not be possible to distinguish between an ordinary walk and a backward walk merely by the load vertically above sensed by the load sensor 70. In this case, the two modes of walking can be distinguished according to the thigh angle Ψ computed by using the inertial sensor 75. Specifically, in an ordinary walk as shown in Fig. 3, the thigh angle Ψ maintains a large positive value in the phases of walking (G) through (A), in which the prosthetic limb 10 is in contact with the ground, but, in a backward walk, the thigh angle Ψ makes a transition from a positive value to a negative value in the phases of walking (E) through (D), in which the prosthetic limb 10 is in contact with the ground. Therefore, the two modes of walking can be distinguished. It is ensured that control to weaken the rotational resistance of the knee shaft 23 is not performed when a backward walk is detected.

When the artificial knee joint 20 is not provided with the load sensor 70, on the other hand, the ground contact sensor 105 can sense whether the foot part 12 is in contact with the ground according to the posture of the respective parts of the prosthetic limb 10 (the foot part 12, the lower leg part 21, the knee shaft 23, the thigh joint part 22, the socket 11, etc.) that can be sensed by referring to the measurement data of the inertial sensor 75 and the knee angle sensor 60 that function as a load sensor and according to geometrical computation based on the length of the artificial knee joint 20 acquired by the length acquisition unit 106. Further, the angle α of forward inclination α of the waist W can be approximately computed from the shin angle Φ, the knee angle θ, the thigh angle Ψ, the length of the artificial knee joint 20 acquired by the length acquisition unit 106, etc. When the inertial sensor 75, which has not detected a load, detects an abrupt acceleration vertical above, it can be assumed that the foot part 12 moving downward hits the ground and comes to a stop, which permits a determination that the artificial knee joint 20 was not in contact with the ground previously. In this case, it is determined that the user walks backward when the thigh angle Ψ makes a transition from a positive value to a negative value, and control of the rotational resistance of the knee shaft 23 to be weaker is prohibited. Alternatively, the rotational resistance of the knee shaft 23 is controlled to be stronger if it has already been weakened.

A description will now be given of specific bend control of the artificial knee joint 20 by the rotational resistance controller 100. As described above with reference to Fig. 3, the rotational resistance controller 100 maintains the knee angle θ to be substantially zero in the Stance phases (A)-(C), by controlling the bending side valve 45 to be less open to strength the bend resistance and restricting the rotation around the knee shaft 23, in order to prevent the artificial knee joint 20 from being bent under the weight of the user. In the Pre-Swing phase (D), the rotational resistance controller 100 controls the bending side valve 45 to be more open to weaken the bend resistance gradually and permit the rotation around the knee shaft 23 so as to make it possible for the artificial knee joint 20 to start bending in preparation for the subsequent Swing phases (E)-(G), which ensures that the knee angle θ has a positive value. In the Swing phases (E)-(G), the rotational resistance controller 100 maintains the bending side valve 45 to be highly open and maintains a state in which the bend resistance is weak and the artificial knee joint 20 is easily bent so as to prevent the prosthetic limb 10 swung away from the ground does not contact the ground.

For the purpose of realizing a smooth walk of the user of the prosthetic limb 10, it is particularly important to cause the bending side valve 45 to make a transition from a less open state to a more open state and to cause the bend resistance to make a transition from a strong state to a weak state in the Mid Stance/Terminal Stance phase (C) or the Pre-Swing phase (D). If the bend resistance is weakened too early, an irregular action of the user of the prosthetic limb 10 such as an abrupt stop increases the likelihood of "knee instability" in which the artificial knee joint 20 that has made a transition to a state of weak bend resistance is bent unintentionally. If the bend resistance is weakened too late, on the other hand, the bend resistance remains strong even in the Initial Swing phase (E) to prohibit the artificial knee joint 20 from being bent so that the likelihood that the prosthetic limb 10 swung away in a stretched state contacts the ground is increased.

The embodiment addresses this by determining the timing of weakening the bend resistance in the Mid Stance/Terminal Stance phase (C) or the Pre-Swing phase (D) according to various criteria listed below. Each of the criteria contributes to resolution of the above issue alone, but the timing of weakening the bend resistance can be optimized by combining these criteria as appropriate.

According to the first criterion, the rotational resistance controller 100 weakens the bend resistance of the knee shaft 23 in accordance with a transition of the thigh angle Ψ computed by the thigh angle computation unit 111 from positive to negative. As shown in Fig. 3, the thigh angle Ψ makes a transition from positive to negative from the Loading Response phase (B) to the Mid Stance/Terminal Stance phase (C). Therefore, the artificial knee joint 20 in which the bend resistance is weakened in association with this can make a smooth transition to the Swing phases (D)-(G). The point of time when the rotational resistance controller 100 starts controlling the bend resistance of the knee shaft 23 to be weaker, i.e., the point of time when the rotational resistance controller 100 starts controlling the bending side valve 45 to be more open, may be a point of time when the thigh angle Ψ decreasing from positive to negative reaches 0 degrees, a point of time when the thigh angle Ψ falls below a negative threshold value, a point of time when the thigh angle Ψ starts to decrease, after a predetermined period of time elapses since the thigh angle Ψ starts to decrease, a point of time when the amount of decrease since the thigh angle Ψ starts to decrease is equal to greater than a predetermined amount, or a point of time when the decrease rate acquired by the thigh angular speed acquisition unit 121 is equal to or greater than a predetermined value.

The rotational resistance controller 100 determines a point of time to start controlling the bend resistance of the knee shaft 23 to be weaker by also allowing for the delay time acquired by the delay time acquisition unit 101. In other words, when the angular speed of the thigh angle Ψ acquired by the thigh angular speed acquisition unit 121 is negative, the rotational resistance controller 100 starts controlling the rotational resistance of the knee shaft 23 to be weaker while the thigh angle Ψ is larger than a threshold value to prevent the thigh angle Ψ from falling below the threshold value during the delay time acquired by the delay time acquisition unit 101. As described above, given, for example, the control period of the rotational resistance controller 100 is 50 ms and the driving period of the motor for opening or closing the bending side valve 45 is 50 ms, a delay time of 100 ms is produced at a maximum. Given that the thigh angle Ψ is +5 degrees and the thigh angular speed is -0.05 degrees/ms, the likelihood that the thigh angle Ψ becomes 0 degrees or negative during the delay time of 100 ms (0.05 degrees/ms×100 ms=5 degrees) is high. Thus, even when the thigh angle Ψ is positive (+5 degrees), the rotational resistance controller 100 starts controlling the bend resistance to be weaker in advance when recognizing the likelihood that the thigh angle Ψ becomes 0 degrees or negative during the delay time, by referring to the negative thigh angular speed (-0.05 degrees/ms) and the delay time (100 ms).

According to the second criterion, the rotational resistance controller 100 weakens the bend resistance of the knee shaft 23 before the knee shaft 23 makes a transition from a point behind the load line L acquired by the load line acquisition unit 103 to a point in front. As shown in Fig. 7, the knee shaft 23 makes a transition from a point behind of the load line L to a point in front of the load line L from the Mid Stance phase (C1) to the Pre-Swing phase (D). Therefore, the artificial knee joint 20 in which the bend resistance is weakened in association with this can make a smooth transition to the Pre-Swing phase (D) and the Swing phases (E)-(G). When the knee shaft 23 is behind the load line L (e.g., the Mid Stance phase (C1)), the artificial knee joint 20 is not bent regardless of the magnitude of the load. On the other hand, when the knee shaft 23 is in front of the load line L (e.g., the Pre-Swing phase (D)), the artificial knee joint 20 is bent provided that the load is greater than the bend resistance. Thus, the bend resistance is weakened, according to the second criterion, before the knee shaft 23 makes a transition from a point behind the load line L to a point in front so that the artificial knee joint 20 can be bent smoothly when the knee shaft 23 reaches a point in front of the load line L.

The point of time when the rotational resistance controller 100 starts controlling the bend resistance of the knee shaft 23 to be weaker, i.e., the point of time when the rotational resistance controller 100 starts controlling the bending side valve 45 to be more open, may be immediately before the knee shaft 23 moving from back to front reaches a point aligned to the load line L, a point of time when the knee shaft 23 starts a forward relative movement with respect to the load line L in the phases of walking (C1)-(D), after a predetermined period of time elapses since the knee shaft 23 starts a forward relative movement with respect to the load line L, a point of time when the amount of relative movement since the knee shaft 23 started a forward relative movement with respect to the load line L is equal to or greater than a predetermined amount, or a point of time when the forward relative speed of the knee shaft 23 with respect to the load line L is equal to or greater than a predetermined value. In consideration of the likelihood that the user of the prosthetic limb 10 loses balance suddenly, it is desired that control is started immediately before the knee shaft 23 reaches a point in front of the load line L. As in the case of the first criterion, the rotational resistance controller 100 may determine a point of time to start controlling the bend resistance of the knee shaft 23 to be weaker by also allowing for the delay time acquired by the delay time acquisition unit 101.

According to the third criterion, the rotational resistance controller 100 weakens the bend resistance of the knee shaft 23 when the first or second criterion is met, and when the angular speed of the thigh angle Ψ and/or the shin angle Φ acquired by the angular speed acquisition unit 120 remains negative for a predetermined period of time continuously. As shown in Fig. 3, the thigh angle Ψ and the shin angle Φ decrease and has a negative angular speed from the Loading Response phase (B) to the Pre-Swing phase (D).

When the thigh angle Ψ and/or the shin angle Φ has a negative angular speed for a predetermined period of time continuously as described above, the likelihood of an ordinary forward walk as shown in Fig. 3 is high so that the rotational resistance controller 100 is permitted to weaken the bend resistance of the knee shaft 23. Conversely, when the thigh angle Ψ or the shin angle Φ does not have a negative angular speed for a predetermined period of time continuously, it is likely that the user is walking in an abnormal manner (e.g., walking backward, jumping, etc.) different from the ordinary walking mode of Fig. 3, so that the user might fall due to knee instability if the bend resistance of the knee shaft 23 is weakened. Therefore, the rotational resistance controller 100 secures the safety of the user of the prosthetic limb 10 by controlling the bending side valve 45 to be less open to strengthen the bend resistance.

According to the fourth criterion, the rotational resistance controller 100 weakens the bend resistance of the knee shaft 23 when the first or second criterion is met and when the thigh angle Ψ and/or the shin angle Φ acquired by the inclination angle acquisition unit 110 at a predetermined time interval has decreased a predetermined number of times consecutively. The fourth criterion, which requires "the thigh angle Ψ and/or the shin angle Φ has decreased a predetermined number of times consecutively" is equivalent to the third criterion, which requires " the angular speed of the thigh angle Ψ and/or the shin angle Φ remains negative for a predetermined period of time continuously".

According to the fifth criterion, the rotational resistance controller 100 weakens the bend resistance of the knee shaft 23 when the knee angle θ measured by the knee angle sensor 60 is equal to or smaller than a predetermined value. As shown in Fig. 3, the knee angle θ is substantially zero in the Mid Stance/Terminal Stance phase (C), in which the bend resistance should be weakened in an ordinary walk on a level ground. When the knee angle θ is equal to or smaller than a predetermined value at a point of time to weaken the bend resistance, the likelihood of an ordinary walk on a level ground is high so that the rotational resistance controller 100 is permitted to weaken the bend resistance of the knee shaft 23.

Conversely, when the knee angle θ is larger than the predetermined value at a point of time to weaken the bend resistance, it is likely that the user is walking on a sloping road, etc., bending the artificial knee joint 20. If the bend resistance of the knee shaft 23 is weakened, the user might fall due to knee instability. Therefore, the rotational resistance controller 100 secures the safety of the user of the prosthetic limb 10 by controlling the bending side valve 45 to be less open to strengthen the bend resistance. The fifth criterion may require, in addition to or in place of the requirement that the knee angle θ is equal or smaller than a predetermined value, that the angular speed of the knee angle θ is equal to or smaller than a predetermined value. During an ordinary walk on a level ground, the angular speed of the knee angle θ is substantially zero in the Stance phases (A)-(C). During a walk on a sloping road, etc. on the other hand, the knee angle θ varies in the Stance phases (A)-(C) and has an angular speed larger than the predetermined value. Therefore, the modes of walking can be distinguished effectively.

According to the sixth criterion, the rotational resistance controller 100 does not control the bend resistance of the knee shaft 23 to be weaker even if any of the above criteria are met, provided that the walking mode sensed by the walking mode sensor 102 is a particular walking mode. More specifically, when an abnormal walking mode (e.g., backward walk, jump, etc.) different from the ordinary walking mode of Fig. 3 and Fig. 7 is sensed, the user might fall due to knee instability if the bend resistance of the knee shaft 23 is weakened. Therefore, the rotational resistance controller 100 secures the safety of the user of the prosthetic limb 10 by controlling the bending side valve 45 to be less open to strengthen the bend resistance.

According to the seventh criterion, the rotational resistance controller 100 strengthen the bend resistance of the knee shaft 23 even if any of the above criteria for weakening the bend resistance is met, provided that the forward movement amount sensed by the forward movement amount sensor 104 makes a transition from a state in which the forward movement amount sensed by the forward movement amount sensor 104 is equal to or larger than a predetermined forward movement amount threshold value to a state in which forward movement amount is smaller than the forward movement amount threshold value. When the user of the prosthetic limb 10 loses speed abruptly in the Terminal Stance phase (C2) of Fig. 7 so that the advancing force Lx is lost, for example, only the load in the vertical direction remains so that the load line L moves behind the knee shaft 23. If the bend resistance of the knee shaft 23 remains weakened in this state, the vertical load might cause knee instability of the artificial knee joint 20, which might lead to a fall of the user. Thus, the rotational resistance controller 100 secures the safety of the user of the prosthetic limb 10 by controlling the bending side valve 45 to be less open to strengthen the bend resistance.

The forward movement amount threshold value can be set as desired. It is preferable that the forward movement amount threshold value be set based on data for the forward movement amount in a past round of walk of the user wearing the artificial knee joint 20. More specifically, the forward movement amount Lx or data indicative thereof that occurs in the Terminal Stance phase (C2) or the Pre-Swing phase (D) of Fig. 7 is measured in multiple rounds of walk under different conditions. The forward movement amount threshold value is set to an arbitrary value smaller than the measured amounts with significance. The bend resistance of the knee shaft 23 may be strengthened by the rotational resistance controller 100 when the amount of decrease (loss) of the forward movement amount sensed by the forward movement amount sensor 104 exceeds a predetermined amount of decrease threshold value, or when the decrease rate (speed of loss) of the forward movement amount sensed by the forward movement amount sensor 104 exceeds a predetermined decrease rate threshold value.

According to the eighth criterion, control by the rotational resistance controller 100 to weaken the bend resistance of the knee shaft 23 is enabled according to the above criteria, provided that the forward movement amount sensed by the forward movement amount sensor 104 is equal to or greater than a predetermined forward movement amount threshold value. In other words, when the forward movement amount Lx of a predetermined amount or greater is sensed, the likelihood of an ordinary forward walk as shown in Fig. 7 is high so that the rotational resistance controller 100 is permitted to weaken the bend resistance of the knee shaft 23.

According to the ninth criterion, control by the rotational resistance controller 100 to weaken the bend resistance of the knee shaft 23 is enabled provided that a state, in which the forward movement amount sensed by the forward movement amount sensor 104 is equal to or greater than a forward movement amount threshold value, continues for a predetermined period of time. The spirit of the ninth criterion is the same as that of the eighth criterion. Requiring that a state in which the forward movement amount Lx equal to or greater than a predetermined amount continues to be sensed for a predetermined period of time makes it more certain that the user is walking forward ordinarily.

As described above, one artificial knee joint 20 constituting the both-leg artificial knee joint device of the present invention has been described. Next, a both-leg artificial knee joint device 200 that performs adjustment and control of two artificial knee joints 20 worn on both legs of the user will be described. When it is necessary to distinguish the artificial knee joints 20 worn on the respective legs of the user from each other, they are referred to as a first artificial knee joint 20A and a second artificial knee joint 20B, respectively, but when it is not necessary to distinguish between them, they are collectively referred to as the artificial knee joint 20.

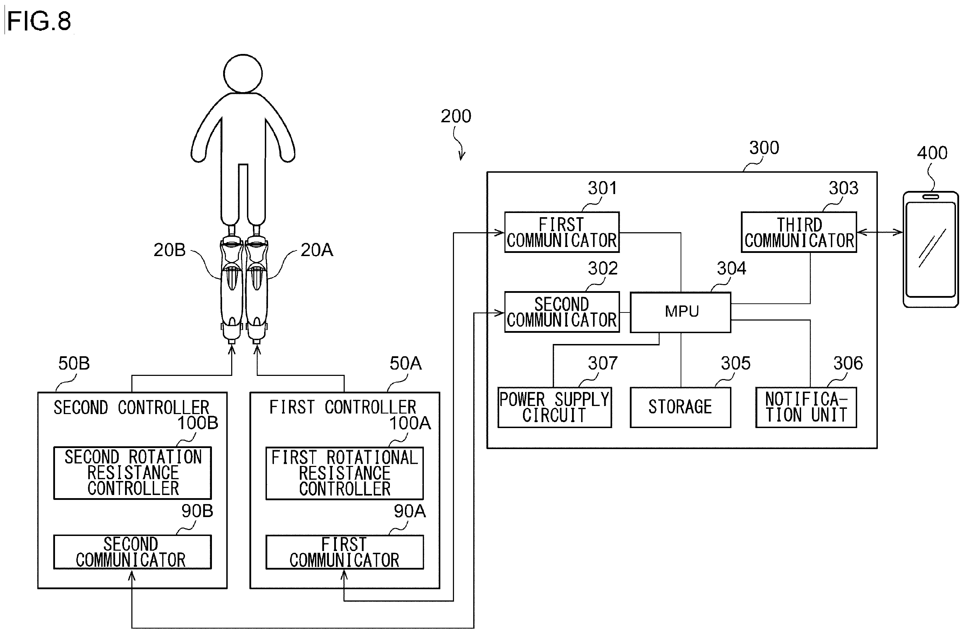

Fig. 8 is a functional block diagram of the both-leg artificial knee joint device 200. The both-leg artificial knee joint device 200 includes the artificial first knee joint 20A worn on a first leg (for example, the left leg) of the user, the second artificial knee joint 20B worn on a second leg (for example, the right leg) of the user, and a communication device 300 capable of communicating with the first artificial knee joint 20A and the second artificial knee joint 20B. The first artificial knee joint 20A and the second artificial knee joint 20B respectively include a first controller 50A and a second controller 50B having functions similar to those of the controller 50 described above. The first controller 50A and the second controller 50B respectively include a first rotational resistance controller 100A and a second rotational resistance controller 100B having functions similar to those of the rotational resistance controller 100 described above, and a first communicator 90A and a second communicator 90B as a transmitter and/or a receiver capable of communicating with the communication device 300.

The communication device 300 is a small communication device that can be carried by a user wearing the artificial knee joints 20 on both legs. The communication device 300 includes a first communicator 301 as a first communication establisher that establishes communication with the first artificial knee joint 90A of the first artificial knee joint 20A to transmit and receive information, a second communicator 302 as a second communication establisher that establishes communication with the second communicator 90B of the second artificial knee joint 20B to transmit and receive information, and a third communicator 303 as a third communication establisher that establishes communication with another communication device 400 such as a smartphone or a tablet to transmit and receive information. In the present embodiment, the first communicator 301, the second communicator 302, and the third communicator 303 communicate in accordance with a short-range wireless communication standard with low power consumption such as Bluetooth (trademark) Low Energy (BLE). Since BLE does not allow simultaneous communication with a plurality of communication targets, a plurality of BLE communicators 301, 302, and 303 are separately provided in the communication device 300, and are individually paired with or connected to the respective communication targets 20A, 20B, and 400.

The communication device 300 further includes a computation processer 304 composed of a micro processor unit (MPU) or the like, a storage 305 composed of a ferroelectric random access memory (FeRAM) or the like, a notification unit 306 composed of a light emitting diode (LED) or the like, and a power supply circuit 307 that supplies power from a battery (not illustrated) to each unit of the communication device 300. The notification unit 306 notifies the remaining amount of the battery, the pairing or connection status of each leg with the artificial knee joint 20 or the other communication device 400, a system error, and the like by light emission of the LED.

The storage 305 stores information or control information for controlling the artificial knee joints 20 of both legs in addition to the information necessary for the operation of the communication device 300. The information stored in the storage 305 may be generated by the other communication device 400 by application software described later and transmitted to the third communicator 303, may be generated by the first artificial knee joint 20A for walking control of the second artificial knee joint 20B based on the state of walk or the like and transmitted to the first communicator 301, or may be generated by the second artificial knee joint 20B for walking control of the first knee joint 20A based on the state of walk or the like and transmitted to the second communicator 302. When the information generated by the other communication device 400, the first artificial knee joint 20A, and the second artificial knee joint 20B is transmitted to the artificial knee joint 20 to be controlled in real time, the information may not be stored in the storage 305.

As described above, the first artificial knee joint 20A and the second artificial knee joint 20B communicate via the communication device 300, and the other communication device 400 communicates with the first artificial knee joint 20A and the second artificial knee joint 20B via the communication device 300. The first artificial knee joint 20A and the second artificial knee joint 20B may directly communicate with each other between the first communicator 90A and the second communicator 90B without passing through the communication device 300. In this case, the information generated by the first artificial knee joint 20A for the walking control of the second artificial knee joint 20B based on the state of walk or the like is directly transmitted from the first communicator 90A to the second communicator 90B, and the information generated by the second artificial knee joint 20B for the walking control of the first artificial knee joint 20A based on the state of walk or the like is directly transmitted from the second communicator 90B to the first communicator 90A.

In addition, some or all of the functions of the communication device 300 that performs communication and control between the artificial knee joints 20 of both legs may be realized by the general-purpose communication device 400 such as a smartphone. When the general-purpose communication device 400 communicates with the artificial knee joint 20 using the BLE, communication cannot be performed simultaneously with the artificial knee joints 20 of both legs due to the limitation of the number of simultaneous connections, but simultaneous connection with the artificial knee joints 20 of both legs may be realized by using another short-range wireless communication standard such as Zigbee (trademark), a mobile communication standard such as 4G or 5G, or another wireless communication standard such as Wi-Fi (trademark). In addition, when the general-purpose communication device 400 communicates with the artificial knee joint 20 using BLE or the like, the artificial knee joint 20 to be connected may be switched in a time division manner according to the phases of walking of the user of the artificial knee joint 20.

The first controller 50A of the first artificial knee joint 20A controls the first artificial knee joint 20A based on information received by the first communicator 90A from the second artificial knee joint 20B and/or the general-purpose communication device 400 via the communication device 300. Similarly, the second controller 50B of the second artificial knee joint 20B controls the second artificial knee joint 20B based on the information received by the second communicator 90B from the first artificial knee joint 20A and/or the general-purpose communication device 400 via the communication device 300. Hereinafter, a specific example of control in which the second artificial knee joint 20B is set as a control target will be described.

In a first control example of the both-leg artificial knee joint device 200, the phases of walking of the second artificial knee joint 20B as the control target is recognized from the phases of walking of the first artificial knee joint 20A as the control subject, and the walking control or the bending and stretching control of the second artificial knee joint 20B is performed. As illustrated in Fig. 3, the phases of walking of one artificial knee joint can be recognized from the phases of walking of the other artificial knee joint. Specifically, when the first artificial knee joint 20A is in the Initial Contact phase (A), the second artificial knee joint 20B is approximately in the Pre-Swing phase (D), when the first artificial knee joint 20A is in the Loading Response phase (B), the second artificial knee joint 20B is approximately in the initial Swing phase (E), when the first artificial knee joint 20A is in the Mid Stance/Terminal Stance phase (C), the second artificial knee joint 20B is approximately in the Mid-Swing phase (F), when the first artificial knee joint 20A is in the Pre-Swing phase (D), the second artificial knee joint 20B is approximately in the Terminal Swing phase (G) to the Initial Contact phase (A), when the first artificial knee joint 20A is in the initial Swing phase (E), the second artificial knee joint 20B is approximately in the Loading Response phase (B), when the first artificial knee joint 20A is in the Mid-Swing phase (F), the second artificial knee joint 20B is approximately in the Mid Stance/Terminal Stance phase (C), and when the first artificial knee joint 20A is in the Terminal Swing phase (G), the second artificial knee joint 20B is approximately in the Pre-Swing phase(D).

As described above with respect to the rotational resistance controller 100, in the walking control or the bending and stretching control of the artificial knee joint 20, it is particularly important to weaken the bend resistance of the artificial knee joint 20 in the transition from the Stance phase to the Swing phase, specifically, in the Mid Stance/Terminal Stance phase (C) or the Pre-Swing phase (D). As described above, when the first artificial knee joint 20A as the control subject is in the Mid-Swing phase (F) or the Terminal Swing phase (G), the second artificial knee joint 20B as the control target is in the Mid Stance/Terminal Stance phase (C) or the Pre-Swing phase (D). Therefore, the first controller 50A transmits walking control information indicating that the first artificial knee joint 20A is in the phases of walking of the Mid-Swing phase (F) or the Terminal Swing phase (G), the second controller 50B that has received the walking control information recognizes that the second artificial knee joint 20B is in the phases of walking of the Mid Stance/Terminal Stance phase (C) or the Pre-Swing phase (D), and the second rotational resistance controller 100B executes walking control to weaken the bend resistance of the second artificial knee joint 20B.

The state that the first artificial knee joint 20A as the control subject is in the phases of walking of the Mid-Swing phase (F) or the Terminal Swing phase (G) can be detected by, for example, that the thigh angle Ψ computed by the thigh angle computation unit 111 is greater than or equal to the positive threshold value, that the angular speed of the thigh angle Ψ acquired by the thigh angular speed acquisition unit 121 is greater than or equal to the positive threshold value, that the shin angle Φ measured by the inertial sensor 75 is greater than or equal to the positive threshold value, that the angular speed of the shin angle Φ acquired by the shin angular speed acquisition unit 122 is greater than or equal to the positive threshold value, that the moment of the lower leg part 21 detected by the load sensor 70 is in the counterclockwise direction in Fig. 3, and the like (see Fig. 3). In the description regarding the rotational resistance controller 100, the first criterion to the ninth criterion regarding the transition condition from the Stance phase to the Swing phase of the artificial knee joint 20 have been described. In addition to or instead of these, the tenth criterion based on the detection information indicating the phases of walking or the state of walk of the other artificial knee joint (first artificial knee joint 20A) as described above may be applied to the walking control or the bending and stretching control of the one artificial knee joint (second artificial knee joint 20B).

In a second control example of the both-leg artificial knee joint device 200, when an abnormal walking mode (e.g., backward walk, jump, etc.) different from the ordinary walking, a walking on a slope, etc. that is different from walking on a level ground, an abrupt loss speed, or the like is detected in the first artificial knee joint 20A as the control subject, control to weaken the bend resistance is not performed or control to strengthen the bend resistance is performed in the second artificial knee joint 20B as the control target. As described above, in a case where the first artificial knee joint 20A does not satisfy the third, fourth, eighth, and ninth criteria relating to the rotational resistance controller 100, or in a case where the first artificial knee joint 20A satisfies the sixth criterion, there is a possibility that an abnormal walking (e.g., backward walk, jump, etc.) different from the ordinary walking mode is performed. Therefore, the second controller 50B that has received the information from the first controller 50A strengthens the bend resistance of the second artificial knee joint 20B by the second rotational resistance controller 100B, thereby ensuring the safety of the user of the both-leg artificial knee joint device 200.

Similarly, in a case where the first artificial knee joint 20A does not satisfy the fifth criterion related to the rotational resistance controller 100, there is a possibility that the user is walking on a slope or the like while bending the first artificial knee joint 20A. Therefore, the second controller 50B that has received the information from the first controller 50A strengthens the bend resistance of the second artificial knee joint 20B by the second rotational resistance controller 100B, thereby ensuring the safety of the user of the both-leg artificial knee joint device 200. In addition, in a case where the first artificial knee joint 20A satisfies the seventh criterion related to the rotational resistance controller 100, there is a possibility that the user of the both-leg artificial knee joint device 200 loses speed abruptly so that the advancing force is lost. Therefore, the second controller 50B that has received the information from the first controller 50A strengthens the bend resistance of the second artificial knee joint 20B by the second rotational resistance controller 100B, thereby ensuring the safety of the user of the both-leg artificial knee joint device 200.

In a third control example of the both-leg artificial knee joint device 200, the first controller 50A as the control subject transmits SOC (State Of Charge) or the like representing the remaining amount of the battery 55 of the first artificial knee joint 20A as information, and the second controller 50B that has received the information adjusts the control period or the like of the second artificial knee joint 20B such that the difference between the remaining amount of the battery 55 of the second artificial knee joint 20B and the remaining amount of the battery 55 of the first artificial knee joint 20A becomes small. For example, when the remaining amount of the battery 55 of the second artificial knee joint 20B is smaller than the remaining amount of the battery 55 of the first artificial knee joint 20A, the second controller 50B lengthens the control period in the second rotational resistance controller 100B or the like, thereby reducing the power consumption in the second artificial knee joint 20B and preventing the battery from running out earlier than the first artificial knee joint 20A.

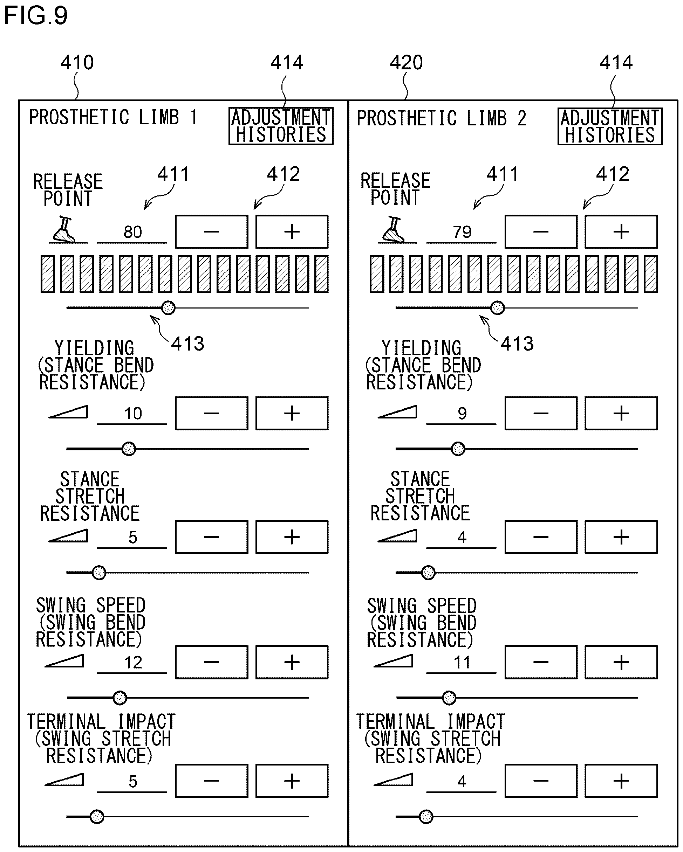

Fig. 9 illustrates a screen example of application software for the both-leg artificial knee joint device 200 executed by the general-purpose communication device 400 such as a smartphone. In the application software, the information on the first artificial knee joint 20A and the second artificial knee joint 20B worn on each leg of the user can be set on the screen and stored in a memory (not illustrated) provided in the storage 305 of the communication device 300 or the controllers 50A and 50B of the artificial knee joints 20A and 20B. In addition, the application software can read information of each of the set artificial knee joints 20A and 20B stored in the memory (not illustrated) provided in the storage 305 of the communication device 300 or the controllers 50A and 50B of each of the artificial knee joints 20A and 20B and display the information on the screen.

An area on the left side of the screen is a first information presentation area 410 for displaying information on the first artificial knee joint 20A (prosthetic limb 1), and an area on the right side of the screen is a second information presentation area 420 for displaying information on the second artificial knee joint 20B (prosthetic limb 2). In each of the information presentation areas 410 and 420, various inputters 411, 412, and 413 capable of inputting each piece of information of the corresponding artificial knee joints 20A and 20B are provided for each piece of information. A numerical value inputter 411 can input information by numerical values, an increase/decrease inputter 412 can increase/decrease the numerical value input to the numerical value inputter 411 by "+" or "-", and a slider inputter 413 can adjust the numerical value input to the numerical value inputter 411 by the slider position. As described above, a presenter is configured such that when inputting the information on one artificial knee joint by each of the inputters 411, 412, and 413, the information of the other artificial knee joint is presented in a juxtaposed manner.

In the illustrated example, five pieces of information of "release point", "yielding (Stance bend resistance)", "Stance stretch resistance", "swing speed (Swing bend resistance)", and "terminal impact (Swing stretch resistance)" are illustrated as the settable information of the artificial knee joints 20A and 20B of each leg. The "release point" is a numerically expressed timing of the transition from the Stance phase to the Swing phase described above in relation to the rotational resistance controller 100, specifically, a timing of weakening the bend resistance of the artificial knee joint 20. In addition, "yielding (Stance bend resistance)" is a numerically expressed value of the bend resistance level of the bending side valve 45 in the Stance phase, "Stance stretch resistance" is a numerically expressed value of the stretch resistance level of the stretching side valve 43 in the Stance phase, "swing speed (Swing bend resistance)" is a numerically expressed value of the bend resistance level of the bending side valve 45 in the Swing phase, and "terminal impact (Swing stretch resistance)" is a numerically expressed value of the stretch resistance level of the stretching side valve 43 in the Swing phase. In addition to or instead of each piece of the above information, when an abnormal walking (e.g., backward walk, jump, etc.) different from the ordinary walking, a walking on a slope, etc. that is different from a walking on a level ground, an abrupt loss of speed, or the like is detected in at least one artificial knee joint, information for strengthening the bend resistance of each artificial knee joint (the bend resistance level of the bending side valve 45) may be set by application software in order to ensure the safety of the user of the both-leg artificial knee joint device 200.

In the screen example as described above, when each piece of information of one artificial knee joint is input by the inputters 411, 412, and 413, each corresponding piece of information of the other artificial knee joint may be input to the inputters 411, 412, and 413 in conjunction with each other. For example, when the "release point" of the first artificial knee joint 20A (prosthetic limb 1) is changed from "80" to "70", the "release point" of the second artificial knee joint 20B (prosthetic limb 2) may be automatically changed from "79" to "69" in conjunction with the "release point". In this case, an adjuster is configured that automatically adjusts, in conjunction with the information of one artificial knee joint input by the inputters 411, 412, and 413, the information of the other artificial knee joint.

In each of the information presentation areas 410 and 420, a history reference unit 414 capable of referring to histories of information of the corresponding artificial knee joint 20A or 20B is further provided. A user of the both-leg artificial knee joint device 200 and an adjustment personnel such as a prosthetist who adjusts the artificial knee joints 20A and 20B of each leg can quickly and accurately readjust the artificial knee joints 20A and 20B by referring to the adjustment histories from the history reference unit 414.

Note that the general-purpose communication device 400 may autonomously refer to the histories of the information of each of the artificial knee joints 20A and 20B stored in the memory inside and outside the general-purpose communication device 400 accessible by the application software, the storage 305 of the communication device 300, the memory (not illustrated) provided in the controllers 50A and 50B of each of the artificial knee joints 20A and 20B, etc., at the time of readjustment of each of the artificial knee joints 20A and 20B, and recommend a numerical value of each information to be input to each of the inputters 411, 412, and 413 on the screen based on the histories. At this time, the general-purpose communication device 400 preferably refers to the occurrence history of the event caused by the insufficient adjustment of the information of each of the artificial knee joints 20A and 20B such as the knee instability, and recommends the numerical value of each information for preventing the recurrence of the event.

As above, the present invention has been described based on the embodiments. The embodiment is intended to be illustrative only and it will be understood by those skilled in the art that various modifications to constituting elements and processes could be developed and that such modifications are also within the scope of the present invention.

In the embodiment, the passive type artificial knee joint in which the rotational resistance of the knee is controlled according to the phases of walking of the user by an electronically controlled hydraulic cylinder or the like has been described, but the present invention is also applicable to an active type artificial knee joint in which the knee is actively bent and stretched by an actuator such as a motor.

The functional configuration of each device described in the embodiments can be achieved by hardware resources or software resources or a cooperation of hardware resources and software resources. Processors, ROMs, RAMs, and other LSIs can be used as hardware resources. Programs such as operating systems and applications can be used as software resources.

Among the embodiments disclosed in the present specification in which a plurality of functions are distributed, some or all of the plurality of functions may be aggregated. Conversely, an aggregation of a plurality of functions may be distributed in part of in the entirety. Regardless of whether functions are aggregated or distributed, the functions may be configured to achieve the purpose of the invention.

Among the embodiments disclosed in the present specification, a configuration including a plurality of objects may integrate the plurality of objects, and conversely, a configuration including one object can be divided into a plurality of objects. The present invention may be configured to achieve an object of the invention regardless of whether or not the present invention is integrated.

The present invention may be expressed as the following items.
6. A both-leg artificial knee joint device (200) comprising:
   a first artificial knee joint (20A) worn on a first leg of a user;
   a second artificial knee joint (20B) worn on a second leg of the user and capable of communicating with the first artificial knee joint (20A); and
   a controller (50) structured to control at least one of the first artificial knee joint (20A) and the second artificial knee joint (20B) based on information received from the other artificial knee joint (20).
7. The both-leg artificial knee joint device (200) according to item 6, further comprising
   a communication device (300) capable of communicating with the first artificial knee joint (20A) and the second artificial knee joint (20B), wherein
   the first artificial knee joint (20A) and the second artificial knee joint (20B) communicate via the communication device (300).
8. The both-leg artificial knee joint device (200) according to item 7, wherein the communication device (300) comprises a first communication establisher structured to establish communication with the first artificial knee joint (20A) and a second communication establisher structured to establish communication with the second artificial knee joint (20B).
9. The both-leg artificial knee joint device (200) according to item 8, wherein
   the communication device (300) comprises a third communication establisher structured to establish communication with another communication device (300), and
   the other communication device (300) communicates with at least one of the first artificial knee joint (20A) and the second artificial knee joint (20B) via the communication device (300).
10. The both-leg artificial knee joint device (200) according to any of items 7 to 9, wherein the communication device (300) comprises a storage (305) structured to store the information.
11. The both-leg artificial knee joint device (200) according to any of items 6 to 10, wherein
   the first artificial knee joint (20A) comprises a first communicator (90A),
   the second artificial knee joint (20B) comprises a second communicator (90B), and
   the first artificial knee joint (20A) and the second artificial knee joint (20B) directly communicate between the first and second communicators.
12. A both-leg artificial knee joint device (200) comprising:
   an inputter capable of inputting first information of a first artificial knee joint (20A) worn on a first leg of a user;
   a presenter structured to present second information of a second artificial knee joint (20B) worn on a second leg of the user when the first information is input by the inputter; and
   a controller (50) structured to control the first artificial knee joint (20A) based on the first information input by the inputter.
13. The both-leg artificial knee joint device (200) according to item 12, wherein the presenter displays the second information side by side with a region to which the first information is capable of being input by the inputter.
14. The both-leg artificial knee joint device (200) according to item 12 or 13, further comprising
   an adjuster structured to adjust the second information presented by the presenter in conjunction with the first information input by the inputter, wherein
   the controller (50) controls the second artificial knee joint (20B) based on the second information adjusted by the adjuster.
15. The both-leg artificial knee joint device (200) according to any of items 12 to 14, further comprising a history reference unit (414) capable of referring to a history of at least one of the first information and the second information.
16. The both-leg artificial knee joint device (200) according to any of items 12 to 15, further comprising a recommender structured to recommend the first information to the inputter based on a history of at least one of the first information and the second information.
19. A method of controlling a both-leg artificial knee joint device (200), the method comprising:
   inputting first information of a first artificial knee joint (20A) worn on a first leg of a user;
   presenting second information of a second artificial knee joint (20B) worn on a second leg of the user when the first information is input in the inputting; and
   controlling the first artificial knee joint (20A) based on the first information input in the inputting.
20. A program for controlling a both-leg artificial knee joint device (200), structured to allow a computer to execute:
   inputting first information of a first artificial knee joint (20A) worn on a first leg of a user;
   presenting second information of a second artificial knee joint (20B) worn on a second leg of the user when the first information is input in the inputting; and
   controlling the first artificial knee joint (20A) based on the first information input in the inputting.

## Claims

1. An artificial knee joint (20) to be worn on one leg of a user, the artificial knee joint (20) comprising
a receiver capable of receiving information from another artificial knee joint (20) worn on another leg of the user; and
a controller (50) structured to control the artificial knee joint (20) based on information received from the other artificial knee joint (20).

2. The artificial knee joint (20) according to claim 1, wherein the information comprises information indicating a state of walk of the other artificial knee joint (20).

3. The artificial knee joint (20) according to claim 1 or 2, wherein the information includes walking control information for controlling bending and stretching of the artificial knee joint (20) according to a phase of walking of the user.

4. The artificial knee joint (20) according to claim 3, wherein the walking control information includes a transition condition from a Stance phase to a Swing phase.

5. The artificial knee joint (20) according to any of claims 1 to 4, wherein
the information includes information indicating a remaining battery amount of the other artificial knee joint (20), and
the controller (50) controls the artificial knee joint (20) so as to reduce a difference between a remaining battery amount of the other artificial knee joint (20) and a remaining battery amount of the artificial knee joint (20).

6. The artificial knee joint (20) according to claim 1, comprising:
a first artificial knee joint (20A) worn on a first leg of a user; and
a second artificial knee joint (20B) worn on a second leg of the user and capable of communicating with the first artificial knee joint (20A); wherein
a controller (50) of at least one of the first artificial knee joint (20A) and the second artificial knee joint (20B) controls its artificial knee joint (20) based on information received from the other artificial knee joint (20) .

7. The artificial knee joint (20) according to claim 6, further comprising
a communication device (300) capable of communicating with the first artificial knee joint (20A) and the second artificial knee joint (20B), wherein
the first artificial knee joint (20A) and the second artificial knee joint (20B) communicate via the communication device (300).

8. The artificial knee joint (20) according to claim 7, wherein the communication device (300) comprises a first communication establisher structured to establish communication with the first artificial knee joint (20A) and a second communication establisher structured to establish communication with the second artificial knee joint (20B).

9. The artificial knee joint (20) according to claim 8, wherein
the communication device (300) comprises a third communication establisher structured to establish communication with another communication device (300), and
the other communication device (300) communicates with at least one of the first artificial knee joint (20A) and the second artificial knee joint (20B) via the communication device (300).

10. The artificial knee joint (20) according to any of claims 7 to 9, wherein the communication device (300) comprises a storage (305) structured to store the information.

11. The artificial knee joint (20) according to any of claims 6 to 10, wherein
the first artificial knee joint (20A) comprises a first communicator (90A),
the second artificial knee joint (20B) comprises a second communicator (90B), and
the first artificial knee joint (20A) and the second artificial knee joint (20B) directly communicate between the first and second communicators.

12. The artificial knee joint (20) according to claim 1, comprising:
an inputter capable of inputting first information of a first artificial knee joint (20A) worn on a first leg of a user;
a presenter structured to present second information of a second artificial knee joint (20B) worn on a second leg of the user when the first information is input by the inputter; and
a controller (50) structured to control the first artificial knee joint (20A) based on the first information input by the inputter.

13. The artificial knee joint (20) according to claim 12, wherein the presenter displays the second information side by side with a region to which the first information is capable of being input by the inputter.

14. The artificial knee joint (20) according to claim 12 or 13, further comprising
an adjuster structured to adjust the second information presented by the presenter in conjunction with the first information input by the inputter, wherein
the controller (50) controls the second artificial knee joint (20B) based on the second information adjusted by the adjuster.

15. The artificial knee joint (20) according to any of claims 12 to 14, further comprising a history reference unit (414) capable of referring to a history of at least one of the first information and the second information.

16. The artificial knee joint (20) according to any of claims 12 to 15, further comprising a recommender structured to recommend the first information to the inputter based on a history of at least one of the first information and the second information.

17. A method of controlling a both-leg artificial knee joint device (200) that comprises a first artificial knee joint (20A) worn on a first leg of a user and a second artificial knee joint (20B) worn on a second leg of the user and capable of communicating with the first artificial knee joint (20A), the method comprising:
transmitting information from one of the first artificial knee joint (20A) and the second artificial knee joint (20B) to the other; and
controlling the other artificial knee joint (20) based on information received from the one artificial knee joint (20) .

18. The method of controlling a both-leg artificial knee joint device (200) according to claim 17, further comprising:
inputting first information of a first artificial knee joint (20A) worn on a first leg of a user;
presenting second information of a second artificial knee joint (20B) worn on a second leg of the user when the first information is input in the inputting; and
controlling the first artificial knee joint (20A) based on the first information input in the inputting.

19. A program for controlling a both-leg artificial knee joint device (200) that comprises a first artificial knee joint (20A) worn on a first leg of a user and a second artificial knee joint (20B) worn on a second leg of the user and capable of communicating with the first artificial knee joint (20A), the program being structured to allow a computer to execute:
transmitting information from one of the first artificial knee joint (20A) and the second artificial knee joint (20B) to the other; and
controlling the other artificial knee joint (20) based on information received from the one artificial knee joint (20) .

20. The program for controlling a both-leg artificial knee joint device (200) according to claim 19, further structured to allow a computer to execute:
inputting first information of a first artificial knee joint (20A) worn on a first leg of a user;
presenting second information of a second artificial knee joint (20B) worn on a second leg of the user when the first information is input in the inputting; and
controlling the first artificial knee joint (20A) based on the first information input in the inputting.
